# FASCICULE DE BREVET EUROPEEN

(11) **EP 1 349 578 B1**
(45) Date de publication et mention de la délivrance du brevet: **26.07.2006**
(21) Numéro de dépôt: 01990621.3
(22) Date de dépôt: 31.12.2001
(51) Int. Cl.: A61K 47/48

(54) **ASSOCIATION MEDICAMENTEUSE D'UNE BIGUANINE (METFORMINE) ET D'ARGININE**
PHARMAZEUTISCHE ZUSAMMENSETZUNG EINES BIGUANIN (METFORMIN) UND ARGININ
MEDICINAL ASSOCIATION OF A BIGUANINE (METFORMIN) AND ARGININE

(30) Priorité: 29.12.2000 FR 0017332
(43) Date de publication de la demande: 08.10.2003
(73) Titulaire: Dospharma, 76130 Mont St Aignan (FR)
(72) Inventeur: RAPIN, Jean-Robert, F-75014 Paris (FR); HALBITTE, Dominique, F-69009 Lyon (FR)
(74) Mandataire: Guerre, Dominique
(86) Numéro de dépôt international: PCT/FR2001/004235
(87) Numéro de publication internationale: WO 2002/053090

(56) Documents cités:
- WO-A-02/12177
- WO-A-99/29314
- WO-A-99/55320
- FR-A- 2 037 002
- FR-A- 2 696 740
- FR-A- 2 796 551
- MARFELLA R ET AL: "Metformin improves hemodynamic and rheological responses to L- arginine in NIDDM patients." DIABETES CARE, (1996 SEP) 19 (9) 934-9., XP001036613

## Description

La présente invention concerne une association médicamenteuse, de deux principes actifs, ayant de manière conjointe une action complémentaire et/ou synergique, et ce pour le traitement du diabète, en particulier du diabète de type 2.

Par "action complémentaire", on entend l'action pharmacologique de deux composés différents permettant d'agir sur la même pathologie par deux mécanismes pharmacologiques respectivement différents, par exemple, l'utilisation combinée de deux antidiabétiques comme une biguanine et une sulfonylurée.

Par "action synergique", on entend l'action pharmacologique de deux composés, consistant àpotentialiser l'action d'au moins un desdits composés, par exemple potentialisation de l'action d'une biguanine ou biguanide (en langue anglaise) par l'action d'un transporteur comme décrit et proposé ci-après dans l'invention.

On sait que la metformine sous forme de chlorhydrate est le médicament de première intention dans le traitement des hyperglycémies et du diabète non insulino-dépendant. Ce chlorhydrate de metformine est utilisé seul ou en association soit avec une sulfonylurée, soit avec un inhibiteur de l'alpha amylase, soit encore avec une glitazone.

Le chlorhydrate de metformine à la dose de 50 mg/kg chez le rat est actif sur les modèles classiques de diabète non insulino-dépendant comme le modèle streptozotocine et le modèle fructose.

Sa biodisponibilité est faible (60%) et son passage intestinal est préférentiel au niveau du jéjunum et de l'iléon. Cette faible biodisponibilité explique l'effet secondaire gênant de la metformine que sont les diarrhées.

La metformine est une biguanine très basique et complètement ionisée aux valeurs du pH intestinal. Son passage implique donc un système physiologique de transporteur, ce qui explique le passage préférentiel.

La présente invention a pour objet de réduire les effets secondaires et d'améliorer la biodisponibilité de la metformine en agissant sur ce système physiologique de transporteur.

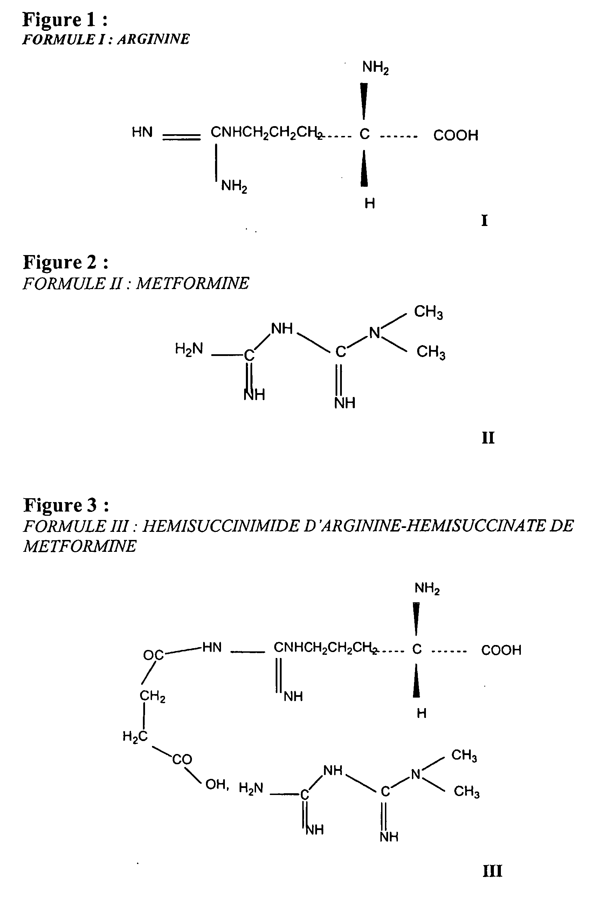

De façon surprenante, la présente invention a mis en évidence, qu'associée à la metformine, l'arginine ayant une analogie structurale avec une biguanine, et en particulier avec une N-diméthyl-biguanine, présente effectivement ce rôle de transporteur vis-à-vis de la metformine. Parmi les transporteurs, l'arginine est préférée en raison de sa similitude de structure chimique avec la metformine. En effet, la L-arginine (cf. fig.3), active elle-même son propre transfert au niveau intestinal. De plus, l'arginine est le précurseur de la synthèse du radical nitroso, NO, qui est reconnu comme un des plus puissants vasodilatateurs, aussi bien au niveau veineux qu'artériel, ainsi que pour ses propriétés hémodynamiques et hémorhéologiques. Cette action propre à l'arginine pourrait être bénéfique sur les pathologies secondaires au développement du diabète, à savoir les macroangiopathies, les microangiopathies, les neuropathies, les néphropathies et les rétinites des diabétiques.

Conformèment au document "Marfella R. et al., Metformin improves hemodynamic and rheological responses to L-arginine in NIDDM patients, Diabetes care, 1996 sep 19(9) 934-9", on a décrit une expérimentation chez l'Homme, en dehors de tout protocole clinique ou thérapeutique, consistant à administrer par voie intraveineuse (sous perfusion) une dose totale de 30 g d'arginine en 30 mn, ce qui est énorme et non faisable en thérapeutique courante.

Conformèment à ce même document, il a été montré que l'administration conjointe de metformine potentialise l'action hémodynamique et hémorhéologique de l'arginine. Mais à l'inverse, les effets thérapeutiques propres à la metformine, en particulier anti-hyperglycémiques, ne sont pas modifiés par l'arginine. Diabetes Care, vol. 19, p. 934 - 939 (Sept 1996), montre l'effet potensialisateur de la metformine conjointe avec L-arginine chez les patients diabétiques. WO-9929314 divulge des sels de metformine avec l'acide fumarique ou succinique pour le traitement du diabète.

Ainsi, les résultats obtenus selon l'invention sur des modèles expérimentaux de diabète ont montré que des composés actifs de metformine comprenant dans leur structure un reste arginine, étaient capables d'augmenter la biodisponibilité de la metformine, de manière inattendue, en potentialisant les effets de celle-ci contre l'hyperglycémie, qui est la manifestation principale du diabète.

Toutes les biguanines actuellement utilisées ou en développement dans le traitement du diabète, présentent les effets secondaires et les problèmes de biodisponibilité cités ci-dessus.

FR 2696740 montre le dérivé de metformine chlorhydrate N-dimethyl N'- diacetyltartroyl biguanide.

La présente invention concerne un ensemble médicamenteux, pour le traitement du diabète, en particulier du diabète de type 2, associant une biguanine, en particulier une N-diméthylbiguanine, en tant que premier médicament, et un agent transporteur de ladite biguanine, en tant que second médicament, le dit ensemble: caractérisé en ce qu'il est un composé actif de formule générale A'---V'---C', capable de restituer au moins la biguanine, par clivage in-vivo de la liaison correspondante entre A' et V', étant spécifié que :
- V est un composé biogénique de vectorisation, de formule générale X-R-Y, dans laquelle,
   * R représente une chaîne hydrocarbonée de 2 à 10 atomes de carbone, aliphatique, cyclique, ou alicyclique, saturée ou non, éventuellement substituée par des groupes alkyles en C1 à C5, et/ou des groupes hydroxyles,
   * X et Y sont chacun une fonction libre acide, amine, ou alcool,
- A est la biguanine et C est l'arginine, A comprend une fonction chimique complémentaire de la fonction X, susceptible de réagir avec cette dernière pour donner une liaison clivable in-vivo, ionique A' --- V' ou covalente A' - V',

C est liée au composé biogénique de vectorisation V pour une reaction d'acylation. L'invention concerne également un composé actif tel que défini ci-dessus, caracterisé en ce que la biguanine A est la metformine.

L'invention concerne un composé actif tel que défini ci-dessus, A étant la metformine, caractérisé en ce que la metformine est liée au composé biogénique de vectorisation, par salification de la fonction amine primaine terminale de la metformine.

L'invention concerne également un composé actif tel que défini ci-dessus, caractérisé en ce que V est choisi parmi l'ensemble des diacides constitué par les acides oxalique, malonique, succinique, glutarique, adipique, pimélique, subérique, azélaique, sébactique, malique, isatique et phtalique et de préférence succinique.

L'invention concerne également un composé actif de formule III

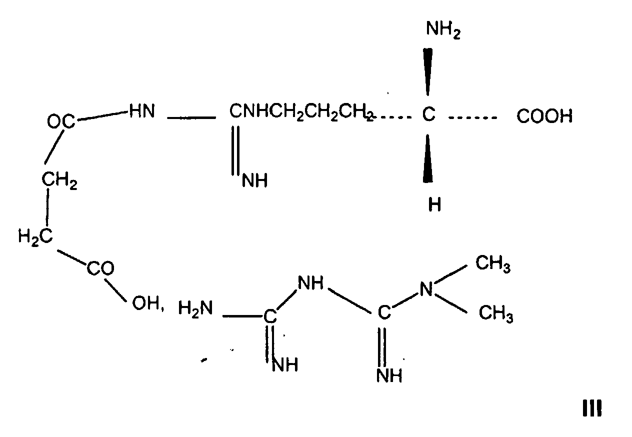

L'invention concerne également l'utilisation en tant que médicament d'un composé tel que précedemment défini.

Elle concerne plus particulièrement l'utilisation en tant que médicament d'un composé de formule générale A'---V'---C' tel que défini précédemment.

L'invention concerne également un procédé de préparation d'un composé actif selon l'invention, comprenant les étapes suivantes :
a) réaction de condensation et/ou salification du composé biogénique de vectorisation V avec la biguanine A
b) réaction de condensation du produit de la réaction selon (a) avec l'arginine C ou a') réaction de condensation du composé biogénique de vectorisation V avec l'arginine C b') réaction de condensation et/ou salification dudit composé biogénique condensé obtenu de la réaction selon (a') avec la biguanine A.

Un autre objet de l'invention est une composition pharmaceutique comprenant au moins un composé selon l'invention, en association avec un ou plusieurs véhicules, diluants, excipients ou adjuvants compatibles et pharmaceutiquement acceptables.

Un autre objet selon l'invention est également une composition pharmaceutique telle que précédemment définie, permettant d'adapter une posologie quotidienne chez l'Homme comprise entre 0,2 g et 1 g dudit composé actif en une ou plusieurs prises.

Dans l'indication anti-diabétique ou anti-hyperglycémique, un ensemble médicamenteux (ou "kit" de traitement thérapeutique) selon l'invention, et en particulier un composé actif de formule A'---V'---C' tel que défini précédemment, peuvent être associés à un autre agent anti-hyperglycémique tel qu'une sulfonylurée.

Par "biguanine" (ou "biguanide"), on entend en particulier les N-diméthylbiguanines, substituées ou non, et par exemple la metformine, mais aussi d'autres composés pharmaceutiques, par exemple la buformine, la fenformine.

Préférentiellement, la biguanine est la metformine.

Par "administration simultanée", on entend l'administration en une seule prise des deux principes actifs, étant entendu que l'administration simultanée permet la libération dans l'organisme des deux principes actifs de manière simultanée ou séquencée.

Par "biogénique", on entend un composé chimique d'origine naturelle ou non et/ou métabolisable, et/ou biodégradable, et/ou atoxique vis à vis de l'Homme ou de l'animal, à dose physiologique.

Par "transporteur" on entend une molécule ou substance qui permet le transfert d'une autre molécule à travers une barrière, soit par formation d'une liaison, soit sans formation de liaison, par activation du système de transport, par exemple par induction protéïque, activation des systèmes oxygéno-dépendants ATPasiques, ou par échange de substance.

Plus particulièrement, par transporteur on entend ici toute molécule ou substance permettant de potentialiser le passage d'une biguanine comme la metformine, et donc d'en faciliter le transport au niveau du jéjunum.

Pour la mise en oeuvre d'une association médicamenteuse telle que précédemment définie, différentes solutions d'administration peuvent être considérées, comme par exemple :
- une formulation ou présentation galénique permettant en une seule dose d'administrer, et la biguanine et le transporteur
- ou deux présentations galéniques respectivement différentes, permettant dans un conditionnement approprié d'administrer en même temps et respectivement, et une dose de bignanine, et une dose du transporteur. cette mise en oeuvre est effectuée en utilisant un composé actif de formule générale A'---V'---C', capable de restituer au moins la biguanine A par clivage in-vivo de la liaison correspondante entre A' et V', étant spécifié que :
- V est un composé biogénique de vectorisation, de formule générale X-R-Y, dans laquelle,
   * R représente une chaîne hydrocarbonée de 2 à 10 atomes de carbone, aliphatique, cyclique, ou alicyclique, saturée ou non, éventuellement substituée par des groupes alkyles en C1 à C5, et/ou des groupes hydroxyles,
   * X et Y sont chacun une fonction libre acide, amine, ou alcool. A est la biguanine et C est l'arginine, A comprend une fonction chimique complémentaire de la fonction X, susceptible de réagir avec cette dernière pour donner une liaison clivable in vivo, ionique A' --- V' ou covalente A' - V', C est lieé au composé biogenigue de vectorisation V par une réaction d'acylation.

On entend ici par "clivage in vivo" toutes les formes d'hydrolyse chimique, susceptibles d'être observées in-vivo, par exemple, hydrolyse acide, et les hydrolyses enzymatiques, par exemple par des amidases ou des estérases.

Par "fonction chimique complémentaire", on entend toute fonction chimique capable de réagir avec une fonction libre ou terminale du composé biogénique. Par exemple, V doit comporter une fonction réagissant avec A (biguanine) et une fonction réagissant avec C (arginine). Ainsi si A et C comportent chacun une fonction acide, V est une diamine, un dialcool ou une alcoolamine, de manière à former respectivement un amide, un ester ou un sel. Ainsi si A et C comportent chacun une fonction amine, V est un diacide de manière à former un amide ou un sel. Si A et C comportent chacun une fonction alcool, V est un diacide de manière à former un diester. Avec ce principe, toutes les compositions sont possibles. En conséquence, si A comporte une fonction acide et C une fonction alcool, V est par exemple une alcoolamine, pour agir avec la fonction acide de A pour donner un amide, un ester ou un sel et avec la fonction alcool de C pour donner un ester.

Par "liaisons covalentes", on entend ici des liaisons chimiques susceptibles d'être formées par réaction de fonctions chimiques dites complémentaires, entre le composé biogénique de vectorisation V et les principes actifs A (biguanine) et C (arginine).

Par "liaisons ioniques", on entend ici des liaisons par force électrostatique, susceptibles d'être formées par action des fonctions chimiques dites complémentaires, entre le composé biogénique de vectorisation V et les principes actifs A (biguanine) et C arginine donc des liaisons du type sels d'acides, sels d'amines, alcoolates et acide/base, et ce indépendamment de la proportion molaire existant entre le composé V et le principe actif A ou C, appartenant au complexe formé par lesdites liaisons ioniques..

Par "liaison clivable in vivo", on entend toute liaison permettant la libération et restititution des principes actifs A biguanine et C (arginine), et du composé biogénique V de vectorisation, in-vivo, par rupture des liaisons ioniques ou covalentes entre les fonctions chimiques complémentaires de A et V.

Les liaisons covalentes clivables sont clivées par action des enzymes présentes dans le milieu in-vivo du site de libération. Ces liaisons covalentes étant des liaisons amides ou des liaisons esters, les enzymes impliquées dans ce clivage sont des amidases, des estérases et des hydrolases. Ces enzymes sont présentes en particulier dans le tube digestif (administration orale), de façon prépondérante dans le foie, dans le sang, et potentiellement présentes dans les organes cibles.

Les amidases qui hydrolysent la liaison -CO-NH- se trouvent au niveau du foie, elles sont peu actives ; d'où un effet prolongé attendu avec le composé selon l'invention porteur d'une telle liaison. Parmi ces amidases, certaines sont connues, il s'agit des endopeptidases qui hydrolysent les liaisons gamma-aminées ou gamma--acides. V peut être en effet selon l'invention un acide gamma-aminé, avec une seconde fonction acide ou amine en position gamma (cas de l'acide glutamique ou de la lysine par exemple).

Les estérases qui hydrolysent la liaison -CO-O- sont très nombreuses dans l'organisme vivant. Elles sont cependant ubiquitaires et très peu spécifiques d'un substrat, d'où une grande vitesse de réaction, avec une libération rapide des constituants A (biguanine), V, C (arginine) du composé actif selon la présente invention. Les plus spécifiques d'un substrat portent le nom de ce substrat, et à ce titre on peut citer par exemple les cholinestérases ou les procaïne-estérases.

Les hydrolases hydrolysent également les esters et toutes les molécules de grande taille apportées à l'organisme sous forme d'aliments. Ces hydrolases sont nombreuses et ubiquitaires également. Elles seront néanmoins spécifiques du composé biogénique de vectorisation V utilisé.

Comme enzymes de clivage utiles pour la mise en oeuvre de la présente invention, on peut citer les enzymes protéolytiques comme la pepsine, la trypsine, les catalases, les endo- et exo- peptidases. Sont également utiles les amylases et les osidases, et enfin les lipases et les béta-oxygènases pour la destruction des lipides.

Ces enzymes n'interviennent que lorsque la structure du composé biogénique de vectorisation comprend une ou plusieurs liaisons qu'elles sont susceptibles de cliver. Par exemple, la lipase agit si le composé biogénique de vectorisation est un diacide de longue chaîne (8 à 10 atomes de carbone, le comparant à un acide gras), et que la liaison A - V ou V - C est obtenue par condensation avec une fonction alcool secondaire de A ou de C.

Les liaisons ioniques clivables sont clivées en fonction de leur lieu de libération, par exemple intestin, foie, plasma, ou organe cible, étant entendu que les sels d'acide, d'amine, ou les alcoolates sont généralement ionisés aux pH des milieux des organismes vivants. Généralement le pH est compris entre 2 et 8, et est par exemple 2 pour l'estomac, et de 6 par exemple pour l'intestin.

On a donc une ionisation du composé actif selon l'invention, en fonction du type de sel utilisé, et une dissociation dudit composé actif, lorsque ce dernier comporte au moins une liaison ionique. On choisit le sel en fonction de sa constante de dissociation, et du pH du site in-vivo de libération. Par exemple pour une dissociation dans l'estomac, on choisit un sel d'acide faible et de base forte.

Le choix du composé biogénique de vectorisation, et notamment le choix de ses fonctions libres X et Y est fait selon la nature des fonctions chimiques, libres et complémentaires, présentes dans ou sur les principes actifs (biguanide et arginine) destinés à être vectorisés, c'est-à-dire liés par liaison covalente ou ionique à ce composé biogénique de vectorisation, mais également selon les sites de clivage et libération choisis. Ce composé biogénique de vectorisation est un produit d'origine naturelle ou non, et/ou métabolisable et/ou biodégradable et/ou atoxique vis-à-vis de l'homme ou de l'animal, à dose physiologique. Ce composé biogénique de vectorisation sera choisi parmi des composés biologiquement éprouvés et décrits, par exemple des gamma-aminoacides intervenant dans la synthèse des protéines, des di-acides intervenant dans le cycle de Kreps, des éthanolamines constitutives de membranes cellulaires, métabolisables et atoxiques, et susceptibles d'être intégrés, eux-mêmes ou leurs métabolites dans les grands cycles biologiques de la vie. On peut citer par exemple à titre de composé biogénique de vectorisation, l'acide succinique que l'on retrouve dans le cycle de Krebs, ou l'acide méthyl-succinique qui est biodégradé en acide succinique.

Les liaisons retenues entre le composé biogénique de vectorisation et les principes actifs associés selon la présente invention, à savoir biguanide et transporteur, dépendent des métabolismes possibles au niveau gastro-intestinal et hépatique.

Par exemple, les sels sont dissociables dans le tube digestif, l'hydrolyse pouvant être retardée par des formes galéniques gastro-résistantes. Les esters sont hydrolysés en milieu acide, ou hydrolysés par les estérases des sucs gastriques, l'hydrolyse pouvant être également retardée par des formes galéniques gastro-résistantes. Les amides sont hydrolysées par les amidases hépatiques, la cinétique de ces hydrolyses étant généralement lente.

Différents tests pour évaluer l'aptitude au clivage in-vivo des liaisons A'---V' et V'---C' et à la libération correspondante des principes actifs A (biguanine) et C (arginine) peuvent être pratiqués. Ces tests consistent par exemple en l'observation de la libération des principes actifs dans un suc intestinal, ou l'étude du métabolisme hépatique sur culture primaire d'hépatocyte de rat. Ces deux tests sont décrits ci-après.

### Essai in-vitro de clivage dans un suc intestinal.

On utilise une préparation de suc intestinal contenant la trypsine, les peptidases, la lipase, l'amylase et toutes les autres enzymes du pancréas exocrine. Ce test est préalablement validé avec des composés étalons. Le composé A'V'C' en quantité connue (de l'ordre du microgramme) est placé en présence d'une quantité connue de suc intestinal (dont les teneurs en trypsine et lipase sont contrôlées). Le mélange réactionnel est maintenu à 37°C pendant une heure. Ce temps est compatible avec le transit intestinal. Des prélèvements sont effectués toutes les 15 mn, et les produits A et C sont détectés et leur concentration est mesurée par HPLC couplée à un détecteur UV, ou un spectromètre de masse s'il n'est pas possible d'utiliser l'UV. Les colonnes utilisées dépendent de la nature de A et de C, mais sont généralement des colonnes échangeuses d'ions, du fait de la présence de formes acides, amines ou alcools libérées. Après étalonnage on détermine la quantité totale de A (biguanine) ou de C (arginine) libérée en 1 heure, et les points intermédiaires permettent de calculer les constantes de dissociation Km et la vitesse Vmax des enzymes pour le composé actif A'V'C' utilisé. Ce test peut être couplé avec une détermination de la libération de A (biguanine), C (arginine) et V dans le suc gastrique, en utilisant exactement le même principe mais en remplaçant le suc intestinal par du suc gastrique.

### Essai in-vitro sur culture primaire d'hépatocytes de rat.

On utilise une culture primaire d'hépatocytes de rat, qui sont proches de ceux de l'Homme pour les études de métabolisme dans un milieu HEPES, à laquelle on ajoute une quantité connue de composé A'V'C' de l'ordre du microgramme. Les produits sont laissés en contact durant 6 heures, et des prélèvements sont effectués à 1 heure, 2 heures et 4 heures, sur lesquels on isole le surnageant et on lyse les hépatocytes du culot. Dans ces milieux, on mesure les concentrations de principes actifs A (biguanine) et C (arginine) libérés. Comme précédemment, le calcul de Vmax et de Km des enzymes impliquées dans le métabolisme est possible.

Lorsque les composés selon l'invention ne traversent pas les membranes cellulaires, le même type d'étude est réalisable sur un homogénat de foie de rat.

L'éventuelle toxicité du composé biogénique de vectorisation est rapportée à celle du composé actif (A'V'C') selon l'invention. Comme ce composé actif est métabolisé en A (biguanine), C (arginine) et V et que V est une substance par définition biologique, la toxicité du composé selon l'invention doit se comparer à la somme des toxicités dues à l'administration de la biguanine A et de l'arginine C. De plus, lorsque le composé actif associe deux principes actifs ayant dans ces conditions , au moins pour un dit principe actif, une efficacité supérieure à celle du même dit principe actif seul, on peut considérer une toxicité moindre pour ledit composé. Néanmoins, une méthode de prédictivité de la toxicité, alternative aux méthodes standards in-vivo, est proposée ci-après pour comparer la toxicité de A et de C et de A'--- V'---C' à des concentrations identiques exprimées en A ou en C (voir Toxicologic Emergencies, Sixth Edition 1997, Goldfranck et al. Appleton and Lange, Connecticut, USA).

### Essai in-vitro de toxicité

Une méthode de culture primaire d'hépatocytes sur 96 heures est mise en oeuvre (voir Biochemical Pharmacology, vol. 50, 1995, pp775-78O). Les hépatocytes sont isolés in situ par une perfusion de collagénase. Ils sont ensuite placés dans un milieu de Williams supplémenté en sérum de veau foetal, en cortisol et en glutamine, à raison de 1 million de cellules par puits. Dans chaque puits on ajoute des concentrations croissantes et toxiques de A (biguanine) et C (arginine et de A' --- V'---C'. Des prélèvements sont effectués après 6h, 1 2h, 24h, 48h et 96h et la viabilité des cellules est déterminée par un test au bleu de méthylène, par l'expression d'albumine, par l'apoptose des hépatocytes, et par la mesure de l'activité des cytocromes P450.

La viabilité des cellules par le test au bleu de méthylène donne des résultats semblables à ceux obtenus par une DL 50.

Les résultats obtenus par l'expression d'albumine permettent de connaître les limites de tolérance de l'hépatocyte à toute substance toxique (toxicité à terme). En effet, un des rôles principaux de l'hépatocyte est de synthétiser des protéines. Lors d'un effet toxique, cette expression de la synthèse et de la libération d'albumine est altérée.

Les résultats obtenus par l'apoptose des hépatocytes permet de confirmer la toxicité à terme, car lors d'un contact avec une substance toxique, les cellules vont programmer leur destruction, ce qui correspond au phénomène d'apoptose qui est mesuré par l'ADN anormal.

La mesure de l'activité des cytocromes P 450 documente les phénomènes d'induction et d'inhibition de ces enzymes, souvent rencontrés avec les produits pharmacologiquement actifs. Une série de tests permet de déterminer l'activité des isoformes des cytocromes P 450.

La présente invention concerne :
- A étant la metformine dans la formule générale précitée, la metformine est liée au compose biogénique de vectorisation V, par salification de la fonction amine primaire terminale de la metformine
- C étant l'arginine dans la formule générale précitée, l'arginine est liée au composé biogénique de vectorisation V par une réaction d'acylation
- V, dans la formule générale précitée, est choisi dans l'ensemble des diacides constitué par les acides oxalique, malonique, succinique, glutarique, adipique, pimélique, subérique, azélaique, sébactique, malique, isatique et phtalique et de préférence succinique.

L'invention concerne aussi, en tant que médicament, le composé de formule III.

L'invention concerne aussi toute composition pharmaceutique comprenant un composé actif tel que précédemment défini, en association avec un ou plusieurs véhicules, diluants, excipients ou adjuvants compatibles et pharmaceutiquement acceptables. Préférentiellement, une telle composition pharmaceutique permet d'adapter une posologie quotidienne chez l'homme, comprise entre 0,2 g et 1 g de chaque principe actif (biguanine et transporteur), en une ou plusieurs prises. Par exemple, des formes galéniques gastrorésistantes peuvent être mises en oeuvre pour éviter toute hydrolyse au niveau de l'estomac.

Préférentiellement, un composé actif tel que défini précédemment peut être obtenu au terme des étapes suivantes :
* réaction de condensation et/ou salification du composé biogénique de vectorisation (V) avec la biguanine A
* réaction de condensation dudit composé biogénique condensé obtenu avec l'arginine C
ou : * réaction de condensation du composé biogenique de vectorisation V avec l'arginine C * réaction de condensation et/ou salification dudit compsé biogenique condensé obtenu avec la biguanine A

Classiquement, les réactions de condensation pouvant être utilisées sont les réactions d'acylation des amines et d'esterification des alcools.

* Lorsque l'un (A ou C) au moins des principes actifs (biguanine ou arginine) est lié au composé biogénique de vectorisation V par une réaction de salification, la séquence de mise en oeuvre des réactions comprendra de préférence la réaction de condensation puis la réaction de salification, pour des raisons de stabilité des sels en fonction du pH, bien connues de l'homme de l'art.

Lorsque A est la metformine, C est l'arginine et V est l'acide succinique, le procédé de préparation comprend les étapes suivantes :
- réaction du monochlorure monoester de l'acide succinique en solution dans de l'éther ou dans du benzène, avec l'arginine en solution aqueuse dans du carbonate de sodium.
- libération de la metformine base à partir du chlorhydrate en milieu sodique concentré et extraction par de l'alcool absolu
- formation du sel de l'hémisuccinimide d'arginine avec la metformine.

De préférence, les compositions pharmaceutiques selon l'invention sont adaptées sous une forme appropriée pour l'administration par voie orale, parentérale ou intraveineuse.

L'invention a plus particulièrement pour objet, l'utilisation d'au moins un composé actif tel que décrit ci-dessus, pour l'obtention de médicaments destinés au traitement du diabète sous toutes ses formes et/ou au traitement des maladies du système circulatoire, que ces maladies soient liées ou non au diabète.

La présente invention est maintenant décrite à titre d'exemple, par référence à l'association de metformine (biguanine) et d'arginine (transporteur) dans un même composé actif A'V'C', V étant l'acide succinique réagissant, d'un côté de manière covalente avec une fonction amine de l'arginine et de l'autre côté de manière ionique (réaction de salifiaction) avec une fonction amine de la metformine.

### Synthèse de l'hémisuccinimide d'arginine-hémisuccinate de metformine

### a) Première étape : préparation de l'hémisuccinimide d'arginine.

De l'arginine base (6g) est dissoute dans 120 ml d'une solution aqueuse de carbonate de sodium (N = 10,6 g/100ml). Par ailleurs, du monochlorure-monoester succinique est dilué dans 50 ml d'éther sulfurique, en léger excès de monochlorure monoester succinique pour une réaction mole à mole par rapport à l'arginine. Sous agitation vigoureuse à température ambiante, la solution éthérée est ajoutée à la solution aqueuse en 10 minutes. Le liquide réactionnel est conservé sous agitation vigoureuse pendant une heure, en chauffant lentement pour une distillation complète de l'éther. On évapore à sec et le résidu est repris par un minimum d'eau distillée (20 ml), et acidifié par de l'acide chlorhydrique dilué. Par concentration ( chauffage léger sous vide partiel) on obtient des cristaux blancs d'hémisuccinimide d'arginine.

La vérification du spectre RMN, de l'analyse centésimale et de la pureté du produit par chromatographie sur couche mince est réalisée. On vérifie en particulier la présence du reste acide aminé de l'arginine par la réaction à la ninhydrine et la présence du carboxyle libre de l'acide succinique par titrimétrie.

Le rendement est quantitatif.

### b) Deuxième étape : libération de la metformine base.

10 grammes de chlorhydrate de metformine sont ajoutés à 40 ml d'une solution d'hydroxyde de sodium 5 N. Le mélange réactionnel est chauffé pendant deux heures à 40°C. Après évaporation sous vide à 40°C, le résidu visqueux est repris par 100 ml d'éthanol absolu. Une filtration permet d' éliminer les impuretés et il reste un résidu non soluble de chlorure de sodium. La metformine base est en solution alcoolique et par évaporation elle est isolée sous forme d'une poudre visqueuse. Le spectre RMN confirme la structure de la metformine. L'absence de chlorure est vérifiée au nitrate d'argent.

On rappelle que la metformine, c'est à dire la N,N-diméthylimidodicarbonimidique diamide, est identifiée dans le MERCK Index sous le numéro 5792 et caractérisée sous le numéro Chemical Abstracts 657-24-9.

### c) Troisième étape

Dans une solution aqueuse d'hémisuccinimide d'arginine, la metformine base est ajoutée mole à mole. Une dissolution immédiate est obtenue.

L'eau est complètement évaporée à 60°C sous vide. Le résidu est remis en solution dans de l'eau distillée et cristallise lors de la concentration sous vide.

On obtient des cristaux translucides solubles dans l'eau et insolubles dans les solvants organiques. Le point de fusion est de 188-189° C.

Le spectre RMN, l'analyse centésimale et la présence d'une seule tache après chromatographie sur couche mince confirme la structure et la pureté du produit. Le rendement total est quantitatif.

A la suite des réactions précédentes,le rendement est voisin de 90%. Les pertes proviennent des purifications et filtrations.

Les formules développées de l'arginine, de la metformine, et du sel de l'hémisuccinimide d'arginine avec la metformine sont données respectivement aux figures 1 à 3.

### Test de clivage :

Ce test est réalisé selon la méthode in-vitro dans un suc intestinal, décrite précédemment selon l'essai in-vitro de toxicité décrit. On observe une libération immédiate de la metformine sans modifier la partie hemisuccinimide-d'arginine. Un deuxième essai est réalisé sur une culture d'hépatocytes de rat, selon la méthode décrite précédemment. On observe une libération lente d'arginine sur 24 heures.

### Toxicité :

Ce test est réalisé selon l'essai in-vitro de toxicité décrit précédemment. La dose toxique est observée avec la metformine à 10⁻² M, et elle est identique pour le composé actif A'-V'-B', à savoir le sel de l'hémisuccinimide d'arginine avec la metformine..

### Vérification de l'activité pharmacologique du composé actif obtenu.

L'intérêt cinétique et pharmacologique du composé actif selon la présente invention est décrit ci-après, en prenant comme exemple illustratif, l'hémisuccinimide d'arginine-hémisuccinate de metformine, et une association chlorhydrate de metformine/chlorhydrate d'arginine :
a) Une étude pharmacocinétique menée dans deux groupes de 20 rats chacun, recevant par voie orale, respectivement 50 mg/kg de chlorhydrate de metformine, et 50 mg/kg d'hémisuccinimide d'arginine-hémisuccinate de metformine a permis de calculer les différents paramètres cinétiques. L'hémisuccinimide d'arginine-hémisuccinate de metformine libère de la metformine et dans les deux groupes, ce sont les taux plasmatiques de la metformine qui sont déterminés.

Après administration de 50 mg/kg de chlorhydrate de metformine, le pic de concentration est observé en 90 minutes et est trouvé égal à 3,9 µg/ml. La fraction biodisponible est de 60% et la demi-vie est égale en moyenne à 2,5 heures.

L'administration de 50 mg/kg d'hémisuccinimide d'arginine-hémisuccinate de metformine correspond à environ 25 mg/kg de chlorhydrate de metformine soit à une demi-dose. Le pic de concentration est observé à 60 minutes et il est trouvé égal à 2,9 µg/ml de metformine. La fraction biodisponible est de 75% et la demi-vie est de 2,6 heures.

Ces résultats démontrent que le passage de la metformine (quantité totale et vitesse de transfert) est amélioré dans le cas de l'hémisuccinimide d'arginine-hémisuccinate de metformine.

Du point de vue pharmacologique, l'activité antidiabétique a été étudiée sur deux modèles de rats rendus diabétiques.

Le premier modèle consiste à traiter les rats par de la streptozotocine (50mg/kg IP), composé qui induit une augmentation de la glycémie qui passe de 5,5 mM à 12-14 mM en 21 jours. L'administration de metformine (30 mg/kg) diminue significativement cette hyperglycémie qui passe de 12,11 à 9,85 mM en moyenne. A la même posologie de 30 mg/kg (environ deux fois moins de metformine base), l'hémisuccinimide d'arginine-hémisuccinate de metformine diminue de façon plus importante l'hyperglycémie qui passe de 12,66 à 7,56 mM. La différence entre les deux traitements est significative en dépit de la plus faible dose de metformine.

Le second modèle est réalisé avec l'administration de fructose à 10% dans l'eau de boisson des rats pendant trois semaines. Il se développe une insulino-résistance, suivie d'un diabète de type non insulino-résistant. L'hémisuccinimide d'arginine-hémisuccinate de metformine se révèle significativement plus actif que la metformine seule à dose équivalente en metformine base et l'action est plus rapide comme montré dans le tableau 1 ci-dessous.

**Tableau 1 :**

| | Metformine 30 mg/kg/jour | Arginine 30 mg/kg/jour | Hémisuccinimide d'arginine-hémisuccinate de metformine 70 mg/kg/jour |
|---|---|---|---|
| Contrôle (glycémie mM) | 6,94 ± 0,13 | 6,87 ± 0,14 | 6,84 ± 0,06 |
| 21 jours fructose (glycémie mM) | 12,52 ± 0,52 | 13,24 ± 0,62 | 12,14 ± 0,28 |
| 4 jours traitement (glycémie mM) | 13,41 ±0,52 | 14,37 ± 0,20 | 10,92 ±0,44 |
| 8 jours traitement (glycémie mM) | 10,83 ± 0,64 | 12,56 ± 0,89 | 9,13 ± 0,64 |
| 12 jours traitement (glycémie mM) | 9,78 ± 0,72 | 11,98 ± 0,71 | 8,43 ± 0,52 |
| 21 jours traitement (glycémie mM) | 9,87 ± 0,68 | 10,73 ±1,27 | 7,76 ± 0,78 |

L'activité antidiabétique a également été vérifiée sur des hamsters rendus diabétiques par l'administration pendant trois mois de fructose. Sur ce modèle l'hémisuccinimide d'arginine-hémisuccinate de metformine se révèle significativement plus actif que la metformine seule à dose égale de 10 mg/kg/jour pour les deux produits après deux semaines de traitement on obtient les résultats illustrés dans le tableau 2 suivant :.

**Tableau 2 :**

| | Fructose 3 mois | Fructose +Metformine 10 mg/kg/jour | Fructose +Hémisuccinimide d'arginine-hémisuccinate de metformine 10 mg/kg/jour |
|---|---|---|---|
| 2 semaines traitement (glycémie mg/dl) | 143 | 112 | 93 |

Glycémie de contrôle sans fructose : 91 mg/dl

Une étude sur la poche jugale du hamster montre que l'hémisuccinimide d'arginine-hémisuccinate de metformine reproduit au moins les effets des deux principes actifs sur la microcirculation, à savoir l'action vasodilatatrice de l'arginine et l'action sur la vasomotion de la metformine.

## Revendications

1. Ensemble médicamenteux, pour le traitement du diabète, en particulier du diabète de type 2, associant une biguanine, en particulier une N-diméthylbiguanine, en tant que premier médicament, et un agent transporteur de ladite biguanine, en tant que second médicament,
- - le dit ensemble comprenant :
a) une quantité thérapeutiquement active de la biguanine
b) et une quantité de l'agent transporteur, prédéterminée pour potentialiser l'activité thérapeutique de la biguanine selon (a).
- chimiquement agencé pour la libération controlés d'au moins la biguanine. **caractérisé en ce qu'**il est un composé actif de formule générale A'---V'---C', capable de restituer au moins la biguanine, par clivage in-vivo de la liaison correspondante entre A' et V', étant spécifié que :
- V est un composé biogénique de vectorisation, de formule générale X-R-Y, dans laquelle,
* R représente une chaîne hydrocarbonée de 2 à 10 atomes de carbone, aliphatique, cyclique, ou alicyclique, saturée ou non, éventuellement substituée par des groupes alkyles en C1 à C5, et/ou des groupes hydrvxyles,
* X et Y sont chacun une fonction libre acide, amine, ou alcool.
- A est la biguanine et C est l'arginine, A comprend une fonction chimique complémentaire de la fonction X, susceptible de réagir avec cette dernière pour donner une liaison clivable in-vivo, ionique A' ― V' ou covalente A' ― V', C et est liée au composé biogénique de vectorisation V par une réaction d'acylation.

2. Composé actif selon la revendication 1, **caractérisée en ce que** V est choisi parmi l'ensemble des diacides constitué par les acides oxalique, malonique, succinique, glutarique, adipique, pimélique, subérique, azélaique, sébactique, malique, isatique et phtalique et de préférence succinique.

3. Composé de formule III

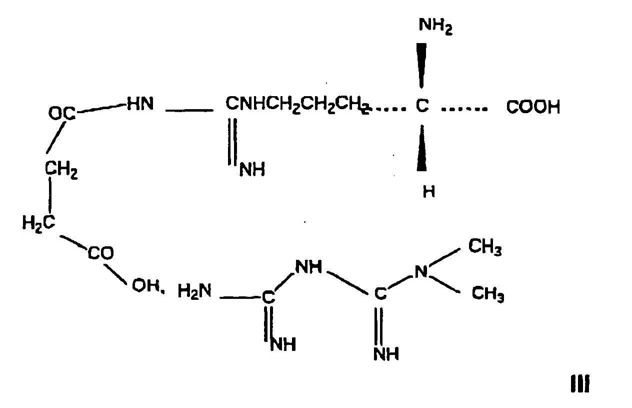

4. Utilisation en tant que médicament d'un composé selon L'une quelconque des revendications 1 à 3.

## Claims

1. Medicinal combination for the treatment of diabetes, in particular of type 2 diabetes, combining a biguanide, in particular an N-dimethylbiguanide, as a first medicament, and an agent for transporting the said biguanide, as a second medicament,
-- said combination comprising:
a) a therapeutically active quantity of the biguanide
b) and a quantity of transporting agent, previously determined to potentiate the therapeutic activity of the biguanide according to (a),
- chemically put together for the controlled release of at least biguanide, **characterized in that** it is an active compound of general formula A'---V'---C', capable of restoring at least the biguanide by cleavage, in vivo, of the corresponding attachment between A' and V', it being specified that:
- V is a biogenic vectorization compound, of general formula X-R-Y, in which,
* R represents an aliphatic, cyclic or alicyclic, saturated or unsaturated hydrocarbon chain of 2 to 10 carbon atoms, which is optionally substituted with C1 to C5 alkyl groups and/or hydroxyl groups,
* X and Y are each a free acid, amine or alcohol function,
- A is biguanide and C is arginine, A comprises a chemical function complementary to the function X, capable of reacting with the latter so as to give an ionic A'---V' or covalent A'―V' attachment which can be cleaved in vivo, and C is attached to the biogenic vectorization compound V using an acylation reaction.

2. Active compound according to Claim 1, **characterized in that** V is chosen from the set of diacids consisting of oxalic, malonic, succinic, glutaric, adipic, pimelic, suberic, azelaic, sebacic, malic, isatic and phthalic acids, and preferably succinic acid.

3. Compound of formula III

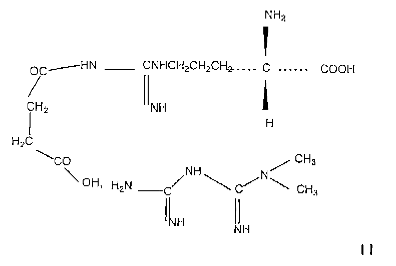

4. Use, as a medicine, of a compound according to any one of Claims 1 to 3.

## Patentansprüche

1. Medizinische Zusammensetzung für die Behandlung von Diabetes, insbesondere Typ-2-Diabetes, in der ein Biguanin, insbesondere ein N-Dimethylbiguanin, als erster Arzneistoff und ein Transoorter für dieses Biguanin als zweiter Arzneistoff kombiniert sind,
- wobei diese Zusammensetzung folgendes umfaßt:
a) eine therapeutisch wirksame Biguaninmenge
b) sowie eine zur Verstärkung der therapeutischen Wirksamkeit des Biguanins gemäß (a) vorbestimmte Transportermenge,
- die chemisch für die kontrollierte Freisetzung von zumindest dem Biguanin eingerichtet ist, **dadurch gekennzeichnet, daß** es sich um einen Wirkstoff der allgemeinen Formel A'==V'==C' handelt, der zumindest das Biguanin durch In-vivo-Spaltung der entsprechenden Bindung zwischen A' und V' wiederherstellen kann, wobei folgendes gilt:
- V ist eine biogene Transportmittelverbindung der allgemeinen Formel X-R-Y, in der
*R eine gesättigte oder ungesättigte a liphatische, cyclische oder alicyclische Kohlenwasserstoffkette mit zwei bis zehn Kohlenstoffatomen bedeutet, die gegebenenfalls durch C1-bis C5-Alkylgruppen und/oder durch Hydroxygruppen substituiert ist,
*X und Y sind jeweils eine freie Säure-, Amin-oder Alkoholfunktion,
- A ist das Biguanin und C das Arginin, A enthält eine chemisch zu der Funktion x komplementäre Funktion, die mit dieser unter Bildung einer in-vivo-spaltbaren ionischen A'-V'-Bindung oder einer kovalenten A'-V'-Bindung reagieren kann und C ist mit der biogenen Transportmittelverbindung V über eine Acylierungsreaktion gebunden.

2. Wirkstoff nach Anspruch 1, **dadurch gekennzeichnet, daß** V aus der Reihe der Dicarbonsäuren Oxalsäure, Malonsäure, Bernsteinsäure, Glutarsäure, Adipinsäure, Pimelinsäure, Korksäure, Azelainsäure, Sebacinsäure, Äpfelsäure, Isatinsäure und Phthalsäure, vorzugswcise Bernsteinsäure, stammt.

3. Verbindung der Formel III

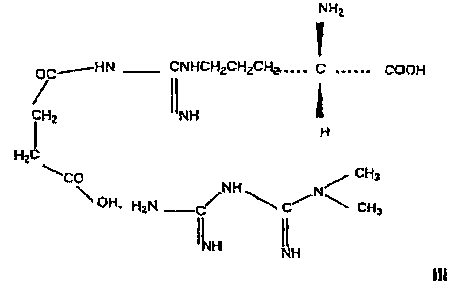

4. Verwendung einer Verbindung nach einem der Ansprüche 1 bis 3 als Arzneimittel.
